# EUROPÄISCHE PATENTSCHRIFT

(11) **EP 0 827 941 B1**
(45) Veröffentlichungstag und Bekanntmachung des Hinweises auf die Patenterteilung: **03.11.1999**
(21) Anmeldenummer: 97250239.7
(22) Anmeldetag: 15.08.1997
(51) Int. Cl.: C03C 10/04, A61K 6/06, C03C 3/078, C03C 3/095

(54) **Sinterbare Lithiumdisilikat-Glaskeramik**
Sinterable lithium disilicate glass-ceramics
Vitrocéramique de disilicate de lithium frittable

(30) Priorität: 05.09.1996 DE 19635940; 06.11.1996 DE 19647739
(43) Veröffentlichungstag der Anmeldung: 11.03.1998
(73) Patentinhaber: IVOCLAR AG, 9494 Schaan (LI)
(72) Erfinder: Schweiger, Marcel, Dipl.-Ing., 7000 Chur (CH); Frank, Martin, Dipl.-Ing., 9494 Schaan (LI); Rheinberger, Volker, Dr., 9490 Vaduz (LI); Höland, Wolfram, Professor Dr., 9494 Schaan (LI)
(74) Vertreter: UEXKÜLL & STOLBERG

(56) Entgegenhaltungen:
- EP-A- 0 690 031
- GB-A- 1 424 398
- US-A- 4 189 325
- US-A- 4 515 634

## Beschreibung

Die Erfindung betrifft sinterbare Lithiumdisilikat-Glaskeramiken und insbesondere solche, die sich aufgrund ihrer Eigenschaften zur Herstellung von geformten Dentalprodukten durch plastische verformung unter Druck- und Wärmeeinwirkung eignen.

Lithiumdisilikat-Glaskeramiken sind aus dem Stand der Technik bekannt. So werden in der EP-B-536 479 selbstglasierte Lithiumdisilikat-Glaskeramikgegenstände beschrieben, die jedoch nicht für dentale Anwendungen vorgesehen sind. Die Glaskeramiken sind frei von La₂O₃ und werden in herkömmlicher Weise durch Erschmelzen geeigneter Ausgangsmaterialien, Eingießen in Formen und anschließende Wärmebehandlung der erhaltenen Gegenstände gebildet.

Auch in der EP-B-536 572 sind Lithiumsilikat-Glaskeramiken offenbart. Durch Aufstreuen eines feinteiligen gefärbten Glases auf ihre Oberfläche erhalten sie Struktur und Färbung, und sie werden als Auskleidungselemente für Bauzwecke eingesetzt. Bei ihrer Herstellung wird in konventioneller Weise vorgegangen, indem geeignete Ausgangsmaterialien erschmolzen, die Schmelze zu einem gewünschten Körper geformt und der Körper zusammen mit aufgestreutem gefärbten Glas wärmebehandelt wird. La₂O₃ ist in der Glaskeramik jedoch nicht enthalten.

Aus der DE-C-1 421 886 sind Glaskeramiken auf der Basis von SiO₂ und Li₂O bekannt, welche große Mengen an physiologisch sehr bedenklichem Arsenoxid enthalten.

Weiter wird im Stand der Technik auch die Verwendung von Lithiumdisilikat-Glaskeramiken in der Dentaltechnik offenbart, wobei diese Glaskeramiken jedoch alle keinerlei La₂O₃ oder MgO enthalten und zu ihrer Verarbeitung zu Dentalprodukten lediglich konventionelle Verfahren eingesetzt werden, bei denen eine Wärmebehandlung zur Ausscheidung von Kristallen nur an homogenen Körpern, nämlich aus einer Glasschmelze geformten Monolithen, wie kleinen Glasblöcken oder -plättchen, durchgeführt wird. Derartige konventionelle Verfahren gestatten jedoch nur, daß eine Volumenkristallisation, nicht jedoch eine Oberflächenkristallisation erfolgt.

Beispiele für solche Glaskeramiken und konventionelle Verfahren zu ihrer Herstellung gehen aus den folgenden Dokumenten hervor.

In US-A-4,515,634 wird eine zur Herstellung von Dentalkronen und -brücken geeignete Lithiumdisilikat-Glaskeramik hoher Festigkeit beschrieben.

Eine hochfeste Lithiumdisilikat-Glaskeramik beschreibt ebenfalls US-A-4,189,325, wobei diese Glaskeramik zwingend CaO zur Fließverbesserung sowie Platin und Nioboxid zur Erzeugung von sehr feinen und einheitlichen Kristallen enthält.

In FR-A-2 655 264 sind Lithiumoxid und Siliciumoxid enthaltende Glaskeramiken zur Herstellung von Dentalprothesen beschrieben, welche sehr große Mengen an MgO enthalten.

Schließlich beschreiben US-A-5,507,981 und WO-A-95/32678 Lithiumdisilikat-Glaskeramiken, die durch spezielle Verfahren zu geformten Dentalprodukten weiterverarbeitet werden können, bei welchen ein Verpressen im viskosen, fließfähigen Zustand bei erhöhten Temperaturen zu dem gewünschten Dentalprodukt erfolgt. Nähere Angaben zu der Herstellung der dabei eingesetzten Plättchen werden nicht gemacht. Auch wird bei der Erzeugung der Glaskeramik in konventioneller Weise vorgegangen, indem homogene Glaskörper, wie zum Beispiel Plättchen, wärmebehandelt werden. Nachteilig an diesen Verfahren ist es, daß sie infolge der Verwendung eines speziellen verpreßbaren Tiegels für einen Zahntechniker sehr aufwendig sind. Weiter werden die glaskeramischen Materialien so weit erhitzt, bis keine Kristalle mehr in dem geschmolzenen Material vorhanden sind, da sonst die Viskosität für das Verpressen zu dem gewünschten Dentalprodukt zu hoch ist. Demnach wird nicht eine Glaskeramik, sondern ein Glas verarbeitet.

Die bekannten Lithiumdisilikat-Glaskeramiken zeigen Unzulänglichkeiten, insbesondere wenn es darum geht, sie im plastischen Zustand zu geformten Dentalprodukten weiterzuverarbeiten. Für eine derartige Verarbeitung ist ihre Viskosität nicht optimal eingestellt, so daß ein kontrolliertes Fließen nicht möglich und die Reaktion mit der eingesetzten Einbettmasse unerwünscht hoch ist. Weiter haben konventionelle Glaskeramiken nur eine geringe Temperaturstandfestigkeit, so daß aus ihnen hergestellte Dentalrestaurationen nur unter Deformation mit einer aufgesinterten Glas- oder Glaskeramik-Schicht versehen werden können. Schließlich mangelt es konventionellen Lithiumdisilikat-Glaskeramiken häufig auch an der erforderlichen chemischen Stabilität für den Einsatz als Dentalmaterial, welches in der Mundhöhle permanent mit Fluiden unterschiedlichster Art umspült wird.

Der Erfindung liegt demgemäß die Aufgabe zugrunde, eine Lithiumdisilikat-Glaskeramik zur Verfügung zu stellen, die ein optimales Fließverhalten bei gleichzeitig geringer Reaktion mit der Einbettmasse bei Verpressen im plastischen Zustand zu Dentalprodukten zeigt, eine hohe Temperaturstandfestigkeit, insbesondere im Bereich von 700 bis 900°C, hat und eine ausgezeichnete chemische Stabilität aufweist.

Diese Aufgabe wird durch die sinterbare Lithiumdisilikat-Glaskeramik nach den Ansprüchen 1 bis 6 gelöst.

Gegenstand der Erfindung sind ebenfalls das Verfahren zur Herstellung von geformten Dentalprodukten nach den Ansprüchen 7 bis 13, die Verwendung der Glaskeramik nach Anspruch 14, geformte Dentalprodukte mit Gehalt an der Glaskeramik nach den Ansprüchen 15 und 16, sowie das Glas nach Anspruch 17.

Die erfindungsgemäße sinterbare Lithiumdisilikat-Glaskeramik ist dadurch gekennzeichnet, daß sie die folgenden Komponenten enthält:

| Komponente | Gew.-% |
|---|---|
| SiO₂ | 57,0 bis 80,0 |
| Al₂O₃ | 0 bis 5,0 |
| La₂O₃ | 0,1 bis 6,0 |
| MgO | 0 bis 5,0, insbesondere 0,1 bis 5,0 |
| ZnO | 0 bis 8,0 |
| K₂O | 0 bis 13,5 |
| Li₂O | 11,0 bis 19,0 |
| P₂O₅ | 0 bis 11,0 |
| Farbkomponenten | 0 bis 8,0 |
| Zusatzkomponenten | 0 bis 6,0 |

wobei

| | |
|---|---|
| (a) Al₂O₃ + La₂O₃ | 0,1 bis 7,0 Gew.-% und |
| (b) MgO + ZnO | 0,1 bis 9,0 Gew.-% |

ausmachen und wobei die Farbkomponenten aus glasfärbenden Oxiden (c) und/oder Farbkörpern (d) in den folgenden Mengen gebildet sind:

| | |
|---|---|
| (c) glasfärbende Oxide | 0 bis 5,0 Gew.-% und |
| (d) Farbkörper | 0 bis 5,0 Gew.-%. |

Vorzugsweise besteht die Glaskeramik im wesentlichen aus den zuvor genannten Komponenten.

Durch Röntgenbeugungsuntersuchungen konnte Lithiumdisilikat als Hauptkristallphase der erfindungsgemäßen Glaskeramik nachgewiesen werden.

Für die einzelnen Komponenten der erfindungsgemäßen Lithiumdisilikat-Glaskeramik existieren bevorzugte Mengenbereiche. Diese können unabhängig voneinander gewählt werden und sind wie folgt:

| Komponente | Gew.-% |
|---|---|
| SiO₂ | 57,0 bis 75,0 |
| Al₂O₃ | 0 bis 2,5 |
| La₂O₃ | 0,1 bis 4,0 |
| MgO | 0,1 bis 4,0 |
| ZnO | 0 bis 6,0, insbesondere 0,1 bis 5,0 |
| K₂O | 0 bis 9,0, insbesondere 0,5 bis 7,0 |
| Li₂O | 13,0 bis 19,0 |
| P₂O₅ | 0 bis 8,0, insbesondere 0,5 bis 8,0 |
| Farbkomponenten | 0,05 bis 6,0 |
| Zusatzkomponenten | 0 bis 3,0. |

Die erfindungsgemäße Glaskeramik enthält vorzugsweise Farbkomponenten, nämlich glasfärbende Oxide (c) und/oder Farbkörper (d), um eine farbliche Anpassung eines aus der Glaskeramik hergestellten Dentalproduktes an das natürliche Zahnmaterial des Patienten zu erzielen. Dabei sorgen die glasfärbenden Oxide, insbesondere TiO₂, CeO₂ und/oder Fe₂O₃, lediglich zum Erhalt einer Farbtönung, wobei die Hauptfärbung durch die Farbkörper hervorgerufen wird. Hierbei ist zu beachten, daß TiO₂ nicht als Keimbildner, sondern in Kombination mit den anderen Oxiden als Farbkomponente wirkt. Als Farbkörper finden bei dentalen Glaskeramiken übliche Metalloxide und insbesondere handelsübliche isochrome Farbkörper, wie z.B. dotierte Spinelle und/oder dotiertes ZrO₂, Anwendung. Bei den Farbkörpern kann es sich sowohl um nicht-fluoreszierende als auch fluoreszierende Materialien handeln.

Neben den zuvor erwähnten Komponenten kann die erfindungsgemäße Lithiumdisilikat-Glaskeramik auch noch Zusatzkomponenten enthalten, wofür insbesondere B₂O₃, F, Na₂O, ZrO₂, BaO und/oder SrO in Frage kommen. Dabei kann mit B₂O₃ und F die Viskosität der Restglasphase der Glaskeramik beeinflußt werden und man nimmt an, daß sie das Verhältnis Oberflächen- zu Volumenkristallisation zugunsten der Oberflächenkristallisation verschieben.

Zur Herstellung der erfindungsgemäßen Glaskeramiken wird insbesondere das im folgenden näher beschriebene Verfahren zur Herstellung von geformten Dentalprodukten mit Gehalt an der Glaskeramik verwendet, wobei spezielle Formgebungen jedoch entfallen können.

Das erfindungsgemäße Verfahren zur Herstellung von geformten Dentalprodukten mit Gehalt an der erfindungsgemäßen sinterbaren Lithiumdisilikat-Glaskeramik zeichnete sich dadurch aus, daß
(a) ein Ausgangsglas, welches die Komponenten gemäß einem der Ansprüche 1 bis 6 mit Ausnahme der Farbkörper enthält, bei Temperaturen von 1200 bis 1650°C erschmolzen wird,
(b) die Glasschmelze unter Bildung eines Glasgranulats in Wasser eingegossen wird,
(c) das Glasgranulat zu einem Pulver mit einer mittleren Korngröße von 1 bis 100 µm, bezogen auf die Teilchenzahl, zerkleinert wird,
(d) dem Pulver die ggf. vorhandenen Farbkörper zugesetzt werden,
(e) das Pulver zu einem Ausgangsglas-Rohling gewünschter Geometrie und heterogener Struktur dichtgepreßt wird, und
(f) der Ausgangsglas-Rohling im Vakuum und im Temperaturbereich von 400 bis 1100°C einer oder mehrerer Wärmebehandlungen unterzogen wird, um ein Dichtsintern zu bewirken und ein als Rohling vorliegendes Dentalprodukt zu bilden.

Im Verfahrensschritt (a) wird ein Ausgangsglas erschmolzen, wozu geeignete Ausgangsmaterialien, wie zum Beispiel Carbonate, Oxide und Fluoride, innig miteinander vermischt und auf die angegebenen Temperaturen erwärmt werden, wodurch sich das Ausgangsglas bildet. Sofern farbgebende Oxide eingesetzt werden sollen, so werden diese dem Gemenge zugegeben. Die Zugabe von gegebenenfalls vorhandenen Farbkörpern erfolgt in einer späteren Stufe des Verfahrens, da ihre Wirkung bei den hohen in der Glasschmelze herrschenden Temperaturen verloren gehen würde.

Sodann wird in Stufe (b) die erhaltene Glasschmelze durch Eingießen in Wasser abgeschreckt und dadurch zu einem Glasgranulat umgewandelt. Diese Vorgehensweise wird üblicherweise auch als Fritten bezeichnet.

Anschließend wird das Glasgranulat in Stufe (c) zerkleinert und insbesondere mit üblichen Mühlen auf die gewünschte Korngröße gemahlen. Dabei ist eine mittlere Korngröße des erhaltenen Pulvers von 10 bis 50 µm, bezogen auf die Teilchenzahl, bevorzugt.

In Stufe (d) erfolgt dann die Zugabe von gegebenenfalls vorhandenen Farbkörpern.

In Stufe (e) wird das Pulver danach zu einem Glasrohling gewünschter Geometrie und heterogener Struktur verdichtet, wobei Preßdrucke von insbesondere 500 bis 2.000 bar Anwendung finden und insbesondere bei Raumtemperatur gearbeitet wird. Dieser Verfahrensschritt des Pressens zu einem Rohling mit heterogener Struktur ist wichtig, damit im Gegensatz zu den aus dem Stand der Technik gekannten Verfahrensweisen bei der folgenden Wärmebehandlung in Stufe (f) neben einer Volumenkristallisation auch eine Oberflächenkristallisation erfolgen kann. So ermöglicht die heterogene Struktur des aus zusammengepreßten Ausgangsglaspulverteilchen bestehenden Ausgangsglas-Rohlings eine gesteuerte Oberflächenkristallisation an den inneren Oberflächen des Glaspulvers. Diese Oberflächenkristallisation ist daran erkennbar, daß auch ohne übliche Volumenkeimbildner, wie zum Beispiel Metalle oder P₂O₅, die in Stufe (f) erfolgende Wärmebehandlung zur Bildung einer fein verteilte Kristalle enthaltenden Lithiumdisilikat-Glaskeramik führt. Bei Verwendung von P₂O₅ als Komponente des Ausgangsglases führt die Wärmebehandlung in Stufe (f) dazu, daß sowohl eine Oberflächenkristallisation als auch eine Volumenkristallisation erfolgt. Bei konventionellen Verfahren werden hingegen Rohlinge eingesetzt, die eine homogene Struktur aufweisen, d.h. bei denen keine Ausgangsglaspulverteilchen vorliegen. Das führt dazu, daß eine Oberflächenkristallisation nicht möglich ist.

Die in Stufe (f) erfolgende Wärmebehandlung dient zu Auslösung der Kristallisation des Ausgangsglas-Rohlings und damit zur Bildung der Glaskeramik, die nach Abschluß dieser Verfahrensstufe als dichtgesinterter glaskeramischer Rohling vorliegt. Dieser Rohling hat üblicherweise die Form eines kleinen Zylinders oder eines kleinen Plättchens.

Zur Erzeugung des endgültigen Dentalproduktes, wie einer Brücke oder einer Krone, bestehen insbesondere die folgenden zwei Möglichkeiten (g1) oder (g2).

Zum einen wird in Stufe (g1) das als Rohling vorliegende Dentalprodukt bei einer Temperatur von 700 bis 1200°C und durch Anwendung von Druck von 2 bis 10 bar zu einem Dentalprodukt gewünschter Geometrie plastisch verformt. Hierzu werden insbesondere das in der EP-A-231 773 beschriebene Verfahren sowie der dort offenbarte Preßofen benutzt. Bei diesem Verfahren wird der Rohling im plastischen Zustand in einen dem gewünschten geformten Dentalprodukt entsprechenden Formhohlraum eingepreßt. Der hierfür eingesetzte Preßofen wird als Empress®-Ofen von der Ivoclar AG, Liechtenstein, vertrieben.

Es wurde festgestellt, daß konventionelle Lithiumdisilikat-Glaskeramiken verschiedenen Anforderungen für eine Weiterverarbeitung zu Dentalprodukten durch plastische Verformung nicht genügen. So ist es für diese Weiterverarbeitung erforderlich, daß der im plastischen Zustand befindliche Rohling in kontrollierter Weise fließt und gleichzeitig nur in geringem Maße mit der Einbettmasse reagiert. Diese beiden Eigenschaften werden bei der erfindungsgemäßen Glaskeramik überraschenderweise durch den Einsatz von La₂O₃ und Al₂O₃ in den angegebenen Mengen erzielt. Daher ist es sehr erstaunlich, daß das als Rohling vorliegende Dentalprodukt fließfähig und im plastischen Zustand verpreßbar ist, obwohl es bereits ein glaskeramisches Material darstellt. Nach dem Stand der Technik werden hingegen stets Gläser als flüssige Schmelze eingesetzt, da sonst ein Verpressen im plastischen Zustand aufgrund zu hoher Viskosität nicht möglich ist.

Es hat sich als besonders vorteilhaft herausgestellt, wenn das als Rohling vorliegende Dentalprodukt eine Viskosität von 10⁵ bis 10⁶ Pa·s bei der plastischen Verformung in Stufe (g1) aufweist.

Weiter kann das als Rohling vorliegende Dentalprodukt auch in Stufe (g2) maschinell zu einem Dentalprodukt gewünschter Geometrie verarbeitet werden, wozu insbesondere computergestützte Fräsmaschinen eingesetzt werden.

In vielen Fällen ist es vorteilhaft, daß das nach Stufe (g1) oder (g2) erhaltene Dentalprodukt gewünschter Geometrie noch in Stufe (h) mit einer Beschichtung versehen wird. Als Beschichtung kommen dabei insbesondere ein Keramik, eine Sinterkeramik, eine Glaskeramik, ein Glas, eine Glasur und/oder ein Composit in Frage. Vorteilhaft sind solche Beschichtungen, die eine Sintertemperatur von 650 bis 950°C und einen linearen thermischen Ausdehnungkoeffizienten haben, welcher kleiner ist als der des zu beschichtenden Dentalproduktes. Besonders vorteilhaft sind Beschichtungen, deren lineare thermische Ausdehnungskoeffizienten nicht mehr als ± 3,0·10⁻⁶K⁻¹ von denen des Substrats abweichen.

Die Aufbringung einer Beschichtung erfolgt insbesondere durch Aufsinterung, zum Beispiel eines Glases, einer Glaskeramik oder eines Composites. Während dieses Sintervorganges wird das Lithiumdisilikat-Glaskeramik enthaltende Dentalprodukt allerdings in einen Temperaturbereich gebracht, der oberhalb des Transformationspunktes der Restglasmatrix der Glaskeramik liegt. Konventionelle Lithiumdisilikat-Glaskeramiken werden hierbei häufig in unerwünschten Weise deformiert, da sie eine zu geringe Temperaturstandfestigkeit haben. Das erfindungsgemäße Dentalprodukt hat jedoch eine ausgezeichnete Temperaturstandfestigkeit, wofür insbesondere der Gehalt an La₂O₃ und Al₂O₃ in den angegebenen Mengen verantwortlich ist.

Neben einer Aufsinterung können auch andere Verfahren angewendet werden, wie sie zum Herstellen von Werkstoffverbunden üblich sind, wie z.B. Kleben oder Löten.

Weiter zeigt die erfindungsgemäße Glaskeramik auch eine sehr gute chemische Beständigkeit, was durch den Einsatz von Al₂O₃, La₂O₃, MgO und ZnO in den angegebenen Mengen hervorgerufen wird.

Neben den zuvor erwähnten Eigenschaften der erfindungsgemäßen Lithiumdisilikat-Glaskeramiken haben diese noch die folgenden weiteren wesentlichen Eigenschaften, durch die sie sich besonders zur Verwendung als Dentalmaterial oder Komponente davon eignen:
- Hohe Biegebruchfestigkeiten von 200 bis 400 MPa. Das Meßverfahren ist in den Beispielen erläutert.
- Hohe Bruchzähigkeiten von 3 bis 4,5 MPa·m^{1/2}. Das Bestimmungsverfahren ist in den Beispielen erläutert.
- Eine mit der des natürlichen Zahnes vergleichbare Transluzenz, obwohl die Erzeugung der erfindungsgemäßen Glaskeramik zumindest teilweise nach dem Mechanismus der Oberflächenkristallisation erfolgt. Das ist deshalb überraschend, da durch Oberflächenkristallisationseffekte oder Initierung der Oberflächenkeimbildung, wie im Falle der Bildung von Oberflächenverspannung durch β-Quarz-Mischkristallbildung, bei anderen Glaskeramiksystemen häufig eine Trübung hervorgerufen wird.
- Anpaßbarkeit der Farbe an die eines natürlichen Zahnes durch Verwendung von Farbkomponenten. Dabei ist erstaunlich, daß trotz der einsetzbaren Farbkomponenten die Festigkeit und Zähigkeit der Glaskeramik nicht nachteilig beeinflußt wird. So ist beispielsweise bei Leucit-Glaskeramiken, die ebenfalls nach dem Mechanismus der Oberflächenkristallisation erzeugt werden, bekannt, daß durch derartige Zusätze die Kristallisation stark beeinflußt und die Festigkeit häufig sehr verringert wird.
- Gute Ätzbarkeit der Glaskeramik, wenn diese als Dentalrestauration eingesetzt wird. Dabei wird zum Beispiel auf der Innenseite einer erfindungsgemäßen Dentalkrone durch kontrolliertes Ätzen ein retentives Muster erzeugt. Bei einem retentiven Muster erfolgt kein flächiger Abtrag der Glaskeramik, wie es zum Beispiel bei Glimmerglaskeramiken der Fall ist, sondern es werden im Oberflächenbereich kleine offenporige Gefüge erzeugt. Durch ein derartiges retentives Muster wird es möglich, daß die Glaskeramik mit Hilfe eines adhäsiven Klebesystemes auf dem natürlichen Zahn befestigt werden kann.

Als erfindungsgemäße geformte Dentalprodukte, die einen Gehalt an der erfindungsgemäßen Glaskeramik aufweisen, kommen insbesondere Dentalrestaurationen, wie zum Beispiel ein Inlay, ein Onlay, eine Brücke, ein Stiftaufbau, eine Verblendung, Schalen, Veneers, Facetten, Verbinder, eine Krone oder eine Teilkrone, in Frage.

Die Erfindung wird nachstehend anhand von Beispielen näher erläutert.

### Beispiele

### Beispiele 1 bis 21

Es wurden insgesamt 21 verschiedene erfindungsgemäße Glaskeramiken und geformte Dentalprodukte mit der in Tabelle I angegebenen chemischen Zusammensetzung hergestellt, indem die Stufen (a) bis (f) des beschriebenen Verfahrens durchgeführt wurden.

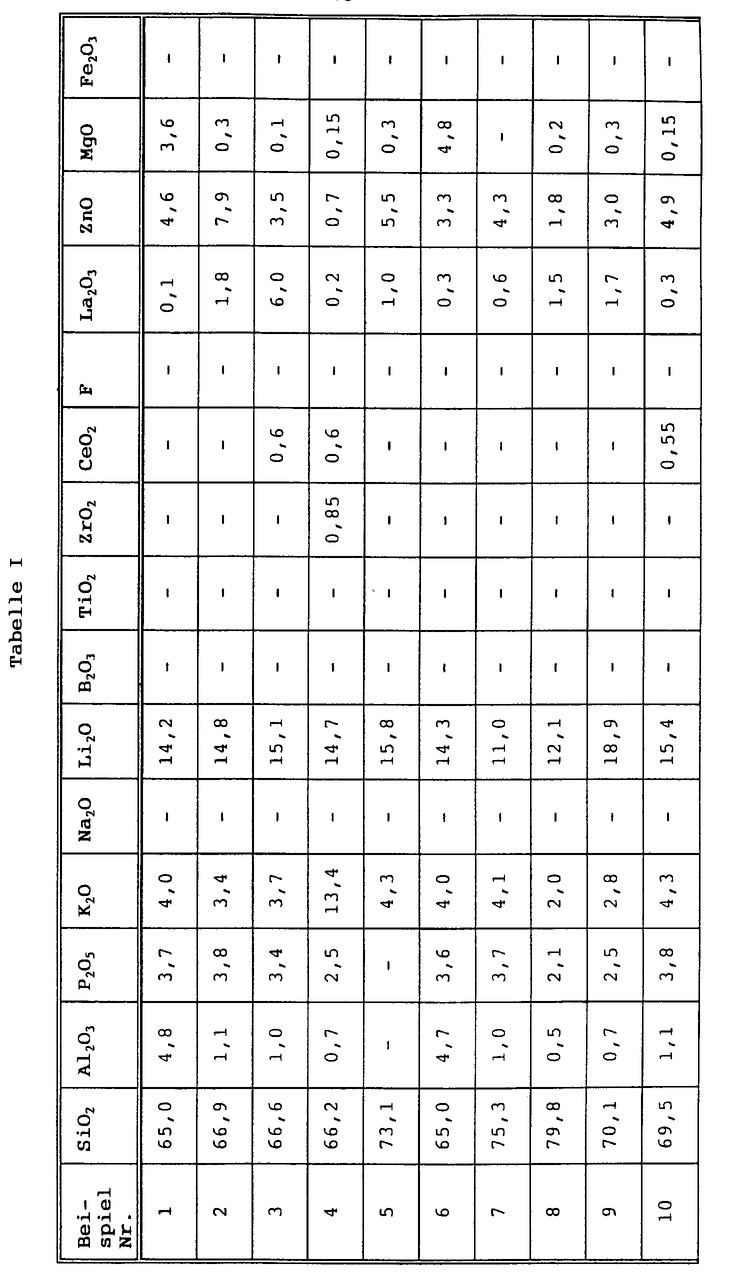

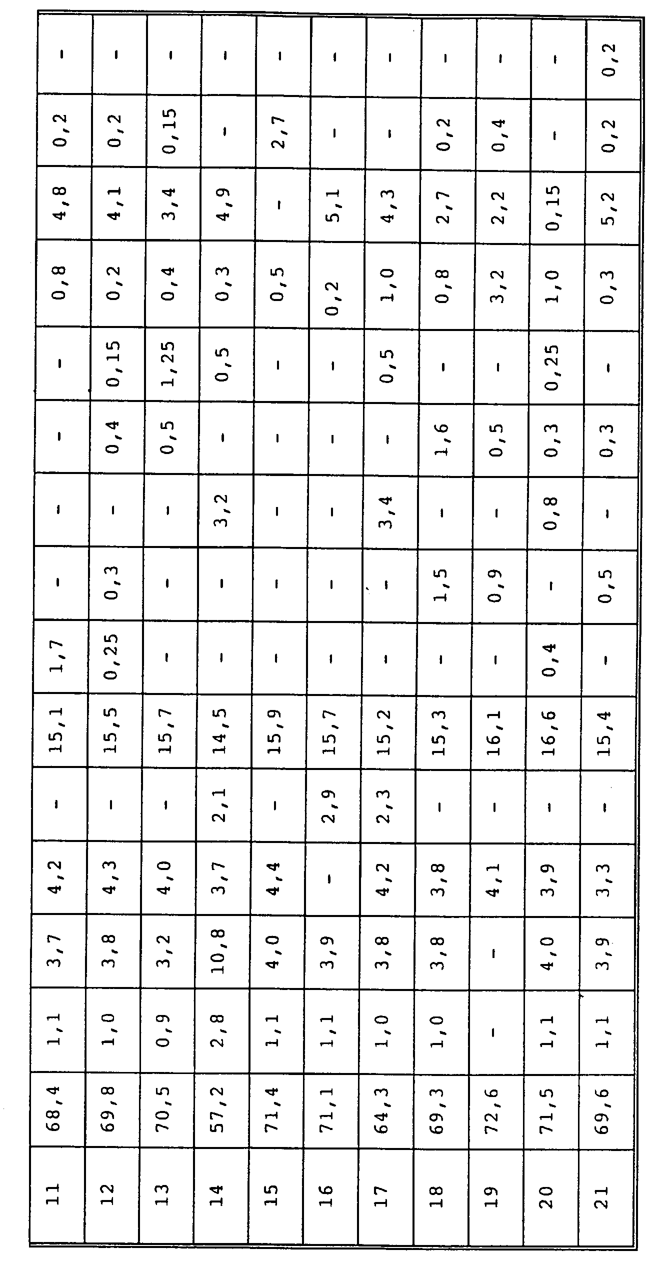

### Beispiel 22

Dieses Beispiel beschreibt die Herstellung einer erfindungsgemäßen Glaskeramik und deren Einsatzmöglichkeit als Gerüstmaterial zur Herstellung eines individuell formbaren vollkeramischen Produktes, wie z.B. eine Krone oder einer mehrgliedrigen Brücke, auf welches zusätzlich eine angepaßte Dentalsinterkeramik aufgebrannt wird.

Zunächst wurde ein Ausgangsglas mit der in Tabelle I, Beispiel 21, angegebenen chemischen Zusammensetzung hergestellt. Dazu wurde ein Gemenge aus Oxiden, Carbonaten und Phosphaten in einem Platin/Rhodium-Tiegel bei einer Temperatur von 1500 bis 1600°C während einer Homogenisierungszeit von einer Stunde erschmolzen. Die Glasschmelze wurde in Wasser abgeschreckt, und die gebildete Glasfritte wurde getrocknet und auf eine mittlere Korngröße von 20 bis 30 µm gemahlen. Durch den Einsatz von glasfärbenden Oxiden, nämlich CeO₂, TiO₂ und Fe₂O₃, konnte auf eine Farbgebung durch Farbkörper verzichtet werden.

Anschließend wurde das eingefärbte Glaspulver mittels einer uniaxialen Trockenpresse bei Raumtemperatur und bei einem Preßdruck von 750 bar zu zylindrischen Ausgangsglasrohlingen, im folgenden als Grünlinge bezeichnet, mit einer Masse von ca. 4 g gepreßt. Die Grünlinge wurden in einem Brennofen unter Vakuum zur erfindungsgemäßen Glaskeramik in Form eines Rohlinges gesintert. In einer ersten Phase wurde der Grünling dazu bei 500°C während einer Stunde vorgebrannt. Dichtgesintert wurde der Rohling dann in einer zweiten Sinterbehandlung bei 850°C während 2 Stunden, wobei mit einer Aufheizrate von 30°C/min. gearbeitet wurde.

### Rohlingeigenschaften

### Optische Eigenschaften

Die erhaltenen Rohlinge wiesen vergleichbare optische Eigenschaften, z.B. Transluzenz, Färbung und Trübung, wie übliche dentalkeramische Verkaufprodukte, wie z.B. IPS Empress OI Rohlinge von IVOCLAR AG, Liechtenstein, auf.

### Biaxialfestigkeit

Zur Bestimmung der Biaxialfestigkeit wurden gesinterte Rohlinge in Scheiben mit einem Durchmesser von 12 mm und einer Dicke von 1,1 mm zersägt. Die Ermittlung der Biaxialfestigkeit erfolgte mit einer Prüfapparatur mit Dreipunktauflage (Stahlkugeln mit einem Durchmesser von 3,2 mm) mit punktueller Krafteinleitung über einen Stempel mit einem Durchmesser von 1,6 mm gemäß ISO 6872-1995 E "Dental Ceramic". Die Vorschubgeschwindigkeit der Lastaufbringung betrug 0,5 mm/min. Die unter diesen Bedingungen ermittelte Biaxialfestigkeit war 261 ± 31 MPa.

Die erhaltenen glaskeramischen Rohlinge wurden schließlich unter Verwendung des Preßverfahrens und Preßofens gemäß EP-A-0 231 773 unter Vakuum im viskosen Zustand in die für den jeweiligen Test gewünschte Probengeometrie verpreßt. Dabei betrugen die Bereitschaftstemperatur des Preßofens 700°C, die Heizrate bis zur Preßtemperatur 60°C/min., die Preßtemperatur 920°C, die Haltezeit bei der Preßtemperatur 10 min. und der Preßdruck 5 bar. Nach dem Preßvorgang wurde die Preßform an der Luft abgekühlt, und die Probenkörper wurden durch Sandstrahlen mit Al₂O₃-Pulver und Glasperlen entformt.

Die erhaltenen Proben hatten folgende Eigenschaften:

### Eigenschaften plastisch verformter Glaskeramik

### Optische Eigenschaften

Die plastisch verformte Glaskeramik hatte transluzente Eigenschaften, die es dem Zahntechniker ermöglichen, aus ihr vollkermaische Dentalprodukte, wie z.B. Kronen oder mehrgliedrige Brücken herzustellen, die optisch den Vorgaben eines natürlichen Zahnes entsprechen. Durch den Einsatz von glasfärbenden Oxiden im Grundglas war die heißgepreßte Glaskeramik zahnfarben getönt. Die Farbintensität konnte dabei durch die Konzentration der färbenden Oxide oder durch den zusätzlichen Einsatz von Farbkörpern gezielt eingestellt werden.

Die Kombination von transluzentem Gerüstmaterial und transluzenter bis transparenter Dentalsinterglaskeramik mit einem Ausdehnungskoeffizienten von 9,1 µm/mK, die schichtweise auf das plastisch geformte Kronen- oder Brückengerüst bei 800°C unter Vakuum aufgesintert wurde, führte zu transluzenten, vollkeramischen Dentalrestaurationen, die den hohen ästhetischen Anforderungen an derartige Produkte genügen.

### 3-Punkt-Biegefestigkeit

Hierzu wurden Stäbe mit den Maßen 1,5 × 4,8 × 20 mm³ gepreßt, und diese wurden mit SiC-Naßschleifpapier (1000-er Körnung) allseitig überschliffen. Die Ermittlung de Biegefestigkeit erfolgte bei einer Stützweite des Prüfmittels von 15 mm und einer Vorschubgeschwindigkeit der Lastaufbringung von 0,5 mm/min. gemäß ISO 6872-1995 E "Dental ceramic". Die bei diesen Bedingungen ermittelte 3-Punkt-Biegefestigkeit betrug 341 ± 98 MPa.

### Linearer thermischer Ausdehnungskoeffizient

Hierzu wurden zylindrische Proben mit einem Durchmesser von 6 mm und einer Länge von 20 mm gepreßt. Der im Temperaturbereich von 100 bis 500°C für diese Proben bestimmte Ausdehnungskoeffizient betrug 10,6 µm/mK.

### Bruchzähigkeit K_{1c}

Hierzu wurden Stäbe mit den Maßen 1,5 × 4,8 × 20 mm³ gepreßt, und diese wurden mit SiC-Naßschleifpapier (1000-er Körnung) allseitig überschliffen. Mit einer Diamanttrennscheibe (0,1 mm Dicke) wurden die Proben einseitig auf eine Tiefe von 2,8 mm gekerbt und anschließend auf ihre 3-Punkt-Biegefestigkeit untersucht. Die Ermittlung der Biegefestigkeit erfolgte bei einer Stützweite des Prüfmittels von 15 mm und einer Vorschubgeschwindigkeit der Lastaufbringung von 0,5 mm/min. Der ermittelte K_{1c}-Wert betrug 4,0 ± 0,2 MPa √m.

### Säurebeständigkeit

Hierzu wurden scheibenförmige Proben mit einem Durchmesser von 15 mm und einer Dicke von 1,5 mm gepreßt und anschließend mit SiC-Naßschleifpapier (1000-er Körnung) allseitig überschliffen. Der gemäß ISO 6872-1995 E "Dental ceramic" bestimmte flächenbezogene Massenverlust dieser Proben wurde nach 16-stündiger Lagerung in 4 Vol.-%iger wäßriger Essigsäurelösung bestimmt, und er betrug lediglich 73 µg/cm² und lag damit deutlich unter dem Normwert für Dentalkeramikmaterialien von 2000 µg/cm².

### Beispiel 23

Dieses Beispiel beschreibt die Herstellung einer erfindungsgemäßen Glaskeramik und deren Einsatzmöglichkeit als Gerüstmaterial zur Herstellung eines individuell formbaren vollkeramischen Produktes, wie z.B einer Krone oder einer mehrgliedrigen Brücke, auf welches zusätzlich eine angepaßte Dentalsinterkeramik aufgebrannt wird.

Zunächst wurde ein Ausgangsglas mit der in Tabelle I, Beispiel 18, angegebenen chemischen Zusammensetzung hergestellt. Dazu wurde ein Gemenge aus Oxide, Carbonaten und Phosphaten in einem Platin/Rhodium-Tiegel bei einer Temperatur von 1500 bis 1600°C während einer Homogenisierungszeit von einer Stunde erschmolzen. Die Glasschmelze wurde in Wasser abgeschreckt, und die gebildete Glasfritte wurde getrocknet und auf eine mittlere Korngröße von 20 bis 30 µm gemahlen. Das Glaspulver wurde mit handelsüblichen Farbkörpern und Fluoreszenzmitteln versetzt und homogenisiert.

Anschließend wurde das eingefärbte Glaspulver mittels einer uniaxialen Trockenpresse bei Raumtemperatur und bei einem Preßdruck von 750 bar zu zylindrischen Grünlingen mit einer Masse von ca. 4 g gepreßt. Die Grünlinge wurden in einem Brennofen unter Vakuum zur erfindungsgemäßen Glaskeramik in Form eines Rohlings gesintert. In einer ersten Phase wurde der Grünling dazu bei 500°C während 20 Minuten vorgebrannt. Dichtgesintert wurde der Rohling dann in einer zweiten Sinterbehandlung bei 850°C während 30 Minuten, wobei mit einer Aufheizrate von 30°C/min. gearbeitet wurde. Zur Bestimmung der Eigenschaften des glaskeramischen Rohlinges wurde, sofern nicht anders angegeben, so wie in Beispiel 22 vorgegangen.

### Rohlingeigenschaften

### Optische Eigenschaften

Die erhaltenen Rohlinge wiesen vergleichbare optische Eigenschaften, wie Transluzenz, Färbung und Trübung, wie übliche dentalkeramische Verkaufsprodukte, z.B. IPS Empress Dentin 24 Rohlinge von IVOCLAR AG, Liechtenstein, auf.

### Biaxialfestigkeit

Die Biaxialfestigkeit betrug 270 ± 38 MPa.

Die erhaltenen glaskeramischen Rohlinge wurden schließlich unter Verwendung des Preßverfahrens und Preßofens gemäß EP-A-0 231 773 unter Vakuum im viskosen Zustand in die für den jeweiligen Test gewünschte Probengeometrie verpreßt. Dabei betrugen die Bereitschaftstemperatur des Preßofens 700°C, die Heizrate bis zur Preßtemperatur 60°C/min., die Preßtemperatur 920°C, die Haltezeit bei der Preßtemperatur 10 min. und der Preßdruck 5 bar. Nach dem Preßvorgang wurde die Preßform an der Luft abgekühlt, und die Probenkörper wurden durch Sandstrahlen mit Al₂O₃-Pulver und Glasperlen entformt.

Die Eigenschaften der erhaltenen Proben wurden gemäß der in Beispiel 22 jeweils beschriebenen Vorgehensweise bestimmt.

### Eigenschaften plastisch verformte Glaskeramik

### Optische Eigenschaften

Die plastisch verformte Glaskeramik hatte transluzente Eigenschaften, die es dem Zahntechniker ermöglichen, aus ihr vollkeramische Dentalprodukte, wie z.B. Kronen oder mehrgliedrige Brücken herzustellen, die optisch den Vorgaben eines natürlichen Zahnes entsprechen. Die Kombination von transluzentem Gerüstmaterial und transluzenter bis transparenter Dentalsinterglaskeramik mit einem Ausdehnungskoeffizienten von 9,1 µm/mK, die schichtweise auf das plastisch geformte Kronen- oder Brückengerüst bei 800°C unter Vakuum aufgesintert wurde, führte zu transluzenten, vollkeramischen Dentalrestaurationen, die den hohen ästhetischen Anforderungen an derartige Dentalprodukte genügen.

### 3-Punkt-Biegefestigkeit

Die 3-Punkt-Biegefestigkeit betrug 347 ± 37 MPa.

### Linearer thermischer Ausdehnungskoeffizient

Der im Temperaturbereich von 100 bis 500°C bestimmte Ausdehnungskoeffizient betrug 10,7 µm/mK.

### Bruchzähigkeit K_{1c}

Der ermittelte K_{1c}-Wert betrug 3,8 ± 0,5 MPa √m.

### Säurebeständigkeit

Der gemäß ISO 6872-1995 bestimmte flächenbezogene Massenverlust nach 16-stündiger Lagerung in 4 Vol.-%iger wäßriger Essigsäurelösung lag deutlich unter dem Normwert für Dentalkeramikmaterialien von 2000 µg/cm².

## Patentansprüche

1. Sinterbare Lithiumdisilikat-Glaskeramik, **dadurch gekennzeichnet**, daß sie die folgenden Komponenten enthält:
| Komponente | Gew.-% |
|---|---|
| SiO₂ | 57,0 bis 80,0 |
| Al₂O₃ | 0 bis 5,0 |
| La₂O₃ | 0,1 bis 6,0 |
| MgO | 0 bis 5,0, insbesondere 0,1 bis 5,0 |
| ZnO | 0 bis 8,0 |
| K₂O | 0 bis 13,5 |
| Li₂O | 11,0 bis 19,0 |
| P₂O₅ | 0 bis 11,0 |
| Farbkomponenten | 0 bis 8,0 |
| Zusatzkomponenten | 0 bis 6,0 |
wobei
| | |
|---|---|
| (a) Al₂O₃ + La₂O₃ | 0,1 bis 7,0 Gew.-% und |
| (b) MgO + ZnO | 0,1 bis 9,0 Gew.-% |
ausmachen und wobei die Farbkomponenten aus glasfärbenden Oxiden (c) und/oder Farbkörpern (d) in den folgenden Mengen gebildet sind:
| | |
|---|---|
| (c) glasfärbende Oxide | 0 bis 5,0 Gew.-% und |
| (d) Farbkörper | 0 bis 5,0 Gew.-%. |

2. Lithiumdisilikat-Glaskeramik nach Anspruch 1, **dadurch gekennzeichnet**, daß sie im wesentlichen aus den angegebenen Komponenten besteht.

3. Lithiumdisilikat-Glaskeramik nach Anspruch 1 oder 2, **dadurch gekennzeichnet**, daß als glasfärbende Oxide TiO₂, CeO₂ und/oder Fe₂O₃ vorhanden sind.

4. Lithiumdisilikat-Glaskeramik nach einem der Ansprüche 1 bis 3, **dadurch gekennzeichnet**, daß sie als Farbkörper dotierten Spinell und/oder dotiertes ZrO₂ enthält.

5. Lithiumdisilikat-Glaskeramik nach einem der Ansprüche 1 bis 4, **dadurch gekennzeichnet**, daß die Zusatzkomponenten B₂O₃, F, Na₂O, ZrO₂, BaO und/oder SrO sind.

6. Lithiumdisilikat-Glaskeramik nach einem der Ansprüche 1 bis 5, **dadurch gekennzeichnet**, daß die Mengen der Komponenten unabhängig voneinander wie folgt sind:
| Komponente | Gew.-% |
|---|---|
| SiO₂ | 57,0 bis 75,0 |
| Al₂O₃ | 0 bis 2,5 |
| La₂O₃ | 0,1 bis 4,0 |
| MgO | 0,1 bis 4,0 |
| ZnO | 0 bis 6,0, insbesondere 0,1 bis 5,0 |
| K₂O | 0 bis 9,0, insbesondere 0,5 bis 7,0 |
| Li₂O | 13,0 bis 19,0 |
| P₂O₅ | 0 bis 8,0, insbesondere 0,5 bis 8,0 |
| Farbkomponenten | 0,05 bis 6,0 |
| Zusatzkomponenten | 0 bis 3,0. |

7. Verfahren zur Herstellung von geformten Dentalprodukten, die die Glaskeramik gemäß einem der Ansprüche 1 bis 6 enthalten, **dadurch gekennzeichnet**, daß
(a) ein Ausgangsglas, welches die Komponenten gemäß einem der Ansprüche 1 bis 6 mit Ausnahme der Farbkörper enthält, bei Temperaturen von 1200 bis 1650°C erschmolzen wird,
(b) die Glasschmelze unter Bildung eines Glasgranulats in Wasser eingegossen wird,
(c) das Glasgranulat zu einem Pulver mit einer mittleren Korngröße von 1 bis 100 µm, bezogen auf die Teilchenzahl, zerkleinert wird,
(d) dem Pulver die ggf. vorhandenen Farbkörper zugesetzt werden,
(e) das Pulver zu einem Ausgangsglas-Rohling gewünschter Geometrie und heterogener Struktur dichtgepreßt wird, und
(f) der Ausgangsglas-Rohling im Vakuum und im Temperaturbereich von 400 bis 1100°C einer oder mehrerer Wärmebehandlungen unterzogen wird, um ein Dichtsintern zu bewirken und ein als Rohling vorliegendes Dentalprodukt zu bilden.

8. Verfahren nach Anspruch 7, **dadurch gekennzeichnet**, daß
(g1) das als Rohling vorliegende Dentalprodukt bei einer Temperatur von 700 bis 1200°C und durch Anwendung von Druck von 2 bis 10 bar zu einem Dentalprodukt gewünschter Geometrie plastisch verformt wird.

9. Verfahren nach Anspruch 7, **dadurch gekennzeichnet**, daß
(g2) das als Rohling vorliegende Dentalprodukt maschinell zu einem Dentalprodukt gewünschter Geometrie verarbeitet wird.

10. Verfahren nach einem der Ansprüche 7 bis 9, **dadurch gekennzeichnet**, daß das Dentalprodukt gewünschter Geometrie
(h) mit einer Beschichtung versehen wird.

11. Verfahren nach Anspruch 10, **dadurch gekennzeichnet**, daß als Beschichtung eine Keramik, eine Sinterkeramik, eine Glaskeramik, ein Glas, eine Glasur und/oder ein Composit eingesetzt wird.

12. Verfahren nach Anspruch 10 oder 11, **dadurch gekennzeichnet**, daß die Beschichtung eine Sintertemperatur von 650 bis 950 °C und einen linearen thermischen Ausdehnungkoeffizienten hat, welcher kleiner ist als der des zu beschichtenden Dentalproduktes.

13. Verfahren nach einem der Ansprüche 10 bis 12, **dadurch gekennzeichnet**, daß die Beschichtung einen linearen thermischen Ausdehnungskoeffizienten hat, welcher nicht mehr als ± 3,0 x 10⁻⁶ K⁻¹ von dem des Dentalprodukts gewünschter Geometrie abweicht.

14. Verwendung der Glaskeramik nach einem der Ansprüche 1 bis 6 als Dentalmaterial oder Komponente von Dentalmaterial.

15. Geformtes Dentalprodukt, welches die Glaskeramik nach einem der Ansprüche 1 bis 6 enthält.

16. Geformtes Dentalprodukt nach Anspruch 15, **dadurch gekennzeichnet**, daß es ein Inlay, ein Onlay, eine Brücke, ein Stiftaufbau, eine Verblendung, eine Krone oder eine Teilkrone ist.

17. Glas, **dadurch gekennzeichnet**, daß es die Komponenten der Glaskeramik gemäß einem der Ansprüche 1 bis 6 mit Ausnahme der Farbkörper enthält.

## Claims

1. Sinterable lithium disilicate glass ceramic, characterized in that it contains the following components:
| Component | % by weight |
|---|---|
| SiO₂ | 57.0 to 80.0 |
| Al₂O₃ | 0 to 5.0 |
| La₂O₃ | 0.1 to 6.0 |
| MgO | 0 to 5.0, in particular 0.1 to 5.0 |
| ZnO | 0 to 8.0 |
| K₂O | 0 to 13.5 |
| Li₂O | 11.0 to 19.0 |
| P₂O₅ | 0 to 11.0 |
| Colour components | 0 to 8.0 |
| Additional components | 0 to 6.0 |
where
| | |
|---|---|
| (a) Al₂O₃ + La₂O₃ make up from | 0.1 to 7.0% by weight and |
| (b) MgO + ZnO make up from | 0.1 to 9.0% by weight |
and the colour components are made up of glass-colouring oxides (c) and/or pigments (d) in the following amounts:
| | |
|---|---|
| (c) glass-colouring oxides | 0 to 5.0% by weight and |
| (d) pigments | 0 to 5.0% by weight. |

2. Lithium disilicate glass ceramic according to Claim 1, characterized in that it consists essentially of the components indicated.

3. Lithium disilicate glass ceramic according to Claim 1 or 2, characterized in that TiO₂, CeO₂ and/or Fe₂O₃ are present as glass-colouring oxides.

4. Lithium disilicate glass ceramic according to any of Claims 1 to 3, characterized in that it contains doped spinel and/or doped ZrO₂ as pigments.

5. Lithium disilicate glass ceramic according to any of Claims 1 to 4, characterized in that the additional components are B₂O₃, F, Na₂O, ZrO₂, BaO and/or SrO.

6. Lithium disilicate glass ceramic according to any of Claims 1 to 5, characterized in that the amounts of the components are, independently of one another, as follows:
| Component | % by weight |
|---|---|
| SiO₂ | 57.0 to 75.0 |
| Al₂O₃ | 0 to 2.5 |
| La₂O₃ | 0.1 to 4.0 |
| MgO | 0.1 to 4.0 |
| ZnO | 0 to 6.0, in particular 0.1 to 5.0 |
| K₂O | 0 to 9.0, in particular 0.5 to 7.0 |
| Li₂O | 13.0 to 19.0 |
| P₂O₅ | 0 to 8.0, in particular 0.5 to 8.0 |
| Colour components | 0.05 to 6.0 |
| Additional components | 0 to 3.0. |

7. Process for producing shaped dental products which contain the glass ceramic according to any of Claims 1 to 6, characterized in that
(a) a starting glass which contains the components as claimed in any of Claims 1 to 6 with the exception of the pigments is melted at temperatures of from 1200 to 1650°C,
(b) the glass melt is poured into water to form a granulated glass,
(c) the granulated glass is comminuted to give a powder having a mean particle size of from 1 to 100 µm, based on the number of particles,
(d) any pigments which are to be present are added to the powder,
(e) the powder is compacted to form a starting glass blank of desired geometry and heterogeneous structure, and
(f) the starting glass blank is subjected to one or more heat treatments under reduced pressure in the temperature range from 400 to 1100°C in order to effect sintering to full density and to form a blank of a dental product.

8. Process according to Claim 7, characterized in that
(g1) the blank of a dental product is plastically formed at a temperature of from 700 to 1200°C by application of a pressure of from 2 to 10 bar to give a dental product.

9. Process according to Claim 7, characterized in that
(g2) the blank of a dental product is machined to give a dental product of desired geometry.

10. Process according to any of Claims 7 to 9, characterized in that the dental product of desired geometry
(h) is provided with a coating.

11. Process according to Claim 10, characterized in that the coating used is a ceramic, a sintered ceramic, a glass ceramic, a glass, a glaze and/or a composite.

12. Process according to Claim 10 or 11, characterized in that the coating has a sintering temperature of from 650 to 950°C and a linear coefficient of thermal expansion which is less than that of the dental product to be coated.

13. Process according to any of Claims 10 to 12, characterized in that the coating has a linear coefficient of thermal expansion which differs from that of the dental product of desired geometry by not more than ± 3.0 x 10⁻⁶ K⁻¹.

14. Use of the glass ceramic according to any of Claims 1 to 6 as dental material or component of dental material.

15. Shaped dental product which contains the glass ceramic as claimed in any of Claims 1 to 6.

16. Shaped dental product according to Claim 15, characterized in that it is an inlay, an onlay, a bridge, a stump build-up, a facing, a crown or a part-crown.

17. Glass, characterized in that it contains the components of the glass ceramic according to any of Claims 1 to 6 with the exception of the pigments.

## Revendications

1. Vitrocéramique frittable à base de disilicate de lithium, caractérisée en ce qu'elle contient les composants suivants:
| Composant | % en poids |
|---|---|
| SiO₂ | 57,0 à 80,0 |
| Al₂O₃ | 0 à 5,0 |
| La₂O₃ | 0,1 à 6,0 |
| MgO | 0 à 5,0, en particulier 0,1 à 5,0 |
| ZnO | 0 à 8,0 |
| K₂O | 0 à 13,5 |
| Li₂O | 11,0 à 19,0 |
| P₂O₅ | 0 à 11,0 |
| Composants colorants | 0 à 8,0 |
| Composants supplémentaires | 0 à 6,0 |
| | |
|---|---|
| (a) Al₂O₃ + La₂O₃ représentant de | 0,1 à 7,0 % en poids et |
| (b) MgO + ZnO représentant de | 0,1 à 9,0 % en poids |
et les composants colorants étant constitués d'oxydes (c) colorant le verre et/ou de corps colorants (d), en les proportions suivantes:
| | |
|---|---|
| (c) Oxydes colorant de verre | 0 à 5,0 % en poids et |
| (d) Corps colorants | 0 à 5,0 % en poids. |

2. Vitrocéramique à base de disilicate de lithium selon la revendication 1, caractérisée en ce qu'elle consiste essentiellement en les composants indiqués.

3. Vitrocéramique à base de disilicate de lithium selon la revendication 1 ou 2, caractérisée en ce que TiO₂, CeO₂ et/ou Fe₂O₃ sont présents en tant qu'oxydes colorant le verre.

4. Vitrocéramique à base de disilicate de lithium selon les revendication 1 à 3, caractérisée en ce qu'elle contient en tant que corps colorants un spinelle dopé et/ou ZrO₂ dopé.

5. Vitrocéramique à base de disilicate de lithium selon l'une des revendications 1 à 4, caractérisée en ce que les composants supplémentaires sont B₂O₃, F, Na₂O, ZrO₂, BaO et/ou SrO.

6. Vitrocéramique à base de disilicate de lithium selon l'une des revendications 1 à 5, caractérisée en ce que les proportions des composants, indépendamment les uns des autres, sont comme suit:
| Composant | % en poids |
|---|---|
| SiO₂ | 57,0 à 75,0 |
| Al₂O₃ | 0 à 2,5 |
| La₂O₃ | 0,1 à 4,0 |
| MgO | 0,1 à 4,0 |
| ZnO | 0 à 6,0, en particulier 0,1 à 5,0 |
| K₂O | 0 à 9,0, en particulier 0,5 à 7,0 |
| Li₂O | 13,0 à 19,0 |
| P₂O₅ | 0 à 8,0, en particulier 0,5 à 8,0 |
| Composants colorants | 0,05 à 6,0 |
| Composants supplémentaires | 0 à 3,0. |

7. Procédé pour la fabrication de produits dentaires façonnés qui contiennent la vitrocéramique selon l'une des revendications 1 à 6, caractérisé en ce que
(a) on fait fondre à des températures de 1 200 à 1 650°C les composants selon l'une des revendications 1 à 6, à l'exception des corps colorants,
(b) on verse dans de l'eau le verre fondu, avec formation d'un verre granulé,
(c) on fragmente le verre granulé en une poudre ayant une taille moyenne de particules de 1 à 100 µm, par rapport au nombre des particules,
(d) on ajoute à la poudre les corps colorants éventuellement présents,
(e) la poudre est compactée en une ébauche de verre de départ à géométrie désirée et structure hétérogène, et
(f) on soumet l'ébauche de verre de départ à un ou plusieurs traitements thermiques dans la plage de températures de 400 à 1 100°C et sous vide, afin de provoquer un frittage dense et de former un produit dentaire présent sous forme d'ébauche.

8. Procédé selon la revendication 7, caractérisé en ce que
(g1) on façonne plastiquement en un produit dentaire de géométrie désirée le produit dentaire présent sous forme d'ébauche, à une température de 700 à 1 200°C et par application d'une pression de 2 à 10 bars.

9. Procédé selon la revendication 7, caractérisé en ce que
(g2) on transforme mécaniquement en un produit dentaire de géométrie désirée le produit dentaire présent sous forme d'ébauche.

10. Procédé selon l'une des revendications 7 à 9, caractérisé en ce que le produit dentaire de géométrie désirée
(h) est muni d'un revêtement.

11. Procédé selon la revendication 10, caractérisé en ce que l'on utilise comme revêtement une céramique, une céramique frittée, une vitrocéramique, un verre, une glaçure et/ou un composite.

12. Procédé selon la revendication 10 ou 11, caractérisé en ce que le revêtement a une température de frittage de 650 à 950°C et un coefficient de dilatation thermique linéaire qui est inférieur à celui du produit dentaire à revêtir.

13. Procédé selon l'une des revendications 10 à 12, caractérisé en ce que le revêtement a un coefficient de dilatation thermique linéaire qui ne s'écarte pas plus de ± 3,0 x 10⁻⁶ K⁻¹ de celui du produit dentaire de géométrie désirée.

14. Utilisation de la vitrocéramique selon l'une des revendications 1 à 6, en tant que matériau dentaire ou composant de matériau dentaire.

15. Produit dentaire façonné, qui contient la vitrocéramique selon l'une des revendications 1 à 6.

16. Produit dentaire façonné selon la revendication 15, caractérisé en ce qu'il s'agit d'un inlay (incrustation), d'un onlay (incrustation de surface), d'un bridge, d'un pivot, d'un revêtement, d'une couronne ou d'une courone partielle.

17. Verre, caractérisé en ce qu'il contient les composants de la vitrocéramique selon l'une des revendications 1 à 6, à l'exception des corps colorants.
